# EUROPEAN PATENT APPLICATION

(11) **EP 4 455 274 A1**
(43) Date of publication of application: **30.10.2024**
(21) Application number: 22899062.8
(22) Date of filing: 24.11.2022
(51) Int. Cl.: C12N 5/078

(54) **ESTABLISHMENT OF ERYTHROID PRECURSOR CELL LINE CAPABLE OF CONDITIONAL MATURATION USING GENE OVEREXPRESSION**

(30) Priority: 26.11.2021 KR 20210165060
(71) Applicant: IUCF-HYU (Industry-University Cooperation Foundation Hanyang University), Seoul 04763 (KR)
(72) Inventor: BAEK, Eun Jung, Seoul 06717 (KR); KIM, Suyeon, Seoul 05545 (KR); JO, Kyeong Won, Changwon-si Gyeongsangnam-do 51618 (KR); PARK, Ju Mi, Changwon-si Gyeongsangnam-do 51747 (KR)
(74) Representative: dompatent von Kreisler Selting Werner - Partnerschaft von Patent- und Rechtsanwälten mbB
(86) International application number: PCT/KR2022/018737
(87) International publication number: WO 2023/096380

(57) **Abstract**

The present invention relates to the establishment of an immortalized erythroid precursor cell line capable of conditional maturation, and more specifically, the present invention provides an erythroid precursor cell for producing an erythrocyte having E6/E7 and Bcl-xL genes transduced therein, and a preparation method therefor.

## Description

### [Technical Field]

The present invention relates to the establishment of an erythroid progenitor cell line capable of conditional maturation using gene overexpression.

### [Background Art]

Blood transfusion is a very important treatment method for patients who lack blood for various reasons. However, blood donated from blood donors has problems such as a shortage of blood supply due to a decrease in the number of people who are able to donate blood and an aging population, the risk of spreading infectious diseases due to blood transfusion, and various other side effects of transfusion. In the case of red blood cell preparations used for the purpose of transporting oxygen among blood components, attempts have been made to use hemoglobin solutions or oxygen carriers as an alternative to the current system of transfusing another person's blood, but the oxygen carrying capacity was low, and serious side effects were shown (Non-Patent Documents 1 and 2). Accordingly, research has been continuously conducted to produce red blood cells (RBC) in vitro (Non-Patent Documents 3 and 4).

RBCs are produced from hematopoietic stem cells through several stages of differentiation: burst forming unit erythroid (BFU-E), colony forming unit erythroid (CFU-E), proerythroblast, basophilic erythroblast, polychromatic erythroblast, and orthochromatic erythroblast, during which the nucleus gradually condenses and the cell size decreases. After the condensed nucleus is enucleated, it becomes a reticulocyte. When the remaining RNA and micro-organelles disappear and both sides become concave, it becomes a mature RBC. This differentiation process generally lasts for three weeks in vitro, and cell proliferation is limited after the stage of the polychromatic erythroblast, which is a mature erythroblast (Non-Patent Document 5).

Hematopoietic stem cells may be isolated from bone marrow, cord blood, and peripheral blood, but their number is limited. Therefore, efforts have been made to produce RBCs from induced pluripotent stem cells or embryonic stem cells, which are theoretically capable of proliferating indefinitely, after differentiating them into hematopoietic stem cells, but they are still at the basic research level (Non-Patent Documents 6 and 7).

Hematopoietic stem cells maintain self-renewal within the human body and produce RBCs and other blood cells throughout one's lifetime, but because they are unable to form an in vivo stem cell niche within the body, they may not maintain self-renewal and they differentiate to produce a limited number of blood cells (Non-Patent Document 8). Therefore, efforts have been made to increase the production of RBCs by inducing the differentiation of hematopoietic stem cells into erythroid cells and extending the cell proliferation period through the regulation of genes related to cell proliferation (Non-Patent Documents 9 to 12). In two previous studies, erythroid progenitor cell lines were established using the human papillomavirus (HPV) viral genes E6/E7. In each study, the established cell lines were named HUDEP and BEL-A. In the case of the HUDEP cell line, the cell line was established, and the proliferation period was extended for about one year. However, although the use of xenogeneic cells must be excluded for future clinical use, in the case of HiDEP, an erythroid progenitor cell line constructed from human induced pluripotent stem cells, OP9 cells, a murine stromal cell line, were used as feeder cells when differentiation is induced (Non-Patent Document 11). Afterward, in the case of the BEL-A cell line established using the same vector in 2017, the cell proliferation period was extended to five months and reticulocytes could be produced. Since the function of the produced reticulocytes was also confirmed, the research is considered to be the most advanced research to date. However, because overexpression of carcinogenic genes called E6 and E7, which are not expressed in humans, was used, there is a safety issue when the nuclei of the final product cannot be completely removed for transfusion (Non-Patent Document 9). In addition, in a study where a cell line was established by suppressing p53 using c-Myc and sox2 genes and short hairpin RNA (shRNA), cell proliferation was maintained for up to one year, but OP9 feeder cells and 10% fetal bovine serum were added to induce enucleation of the cells (Non-Patent Document 12). In a study where a cell line was established using a combination of Bcl-XI, and c-Myc, the genes were transduced into CD71-positive erythroblasts to extend cell proliferation for up to eight months, but almost no enucleation of the cells was achieved (Non-Patent Document 13). Most studies have established erythroid progenitor cell lines and extended cell proliferation, but there are problems such as safety issues due to the use of genes that are not present in the human body, very low efficiency when transducing a gene into hematopoietic stem cells/erythroid progenitor cells, and cell maturation not being achieved in serum-free media.

### [Related Art Documents]

### [Non-Patent Documents]

1. Natanson C et al. JAMA. 2008 May 21;299(19):2304-12.
2. Spahn DR. Crit Care. 1999;3(5):R93-7.
3. Migliaccio AR et al. Blood Rev. 2012 Mar;26(2):81-95.
4. Bouhassira EE. Stem Cells Transl Med. 2012 Dec;1(12):927-33.
5. Hu JP et al. Blood. 2013 Apr 18;121(16):3246-53.
6. Lu SJ et al. Blood. 2008 Dec 1;112(12):4475-84.
7. Dorn I et al. Haematologica. 2015 Jan;100(1):32-41.
8. Glettig DL et al. Biores Open Access. 2013 Jun;2(3):179-85.
9. Trakarnsanga K et al. Nat Commun. 2017 Mar 14;8:14750.
10. Hirose S et al. Stem Cell Reports. 2013;1(6):499-508.
11. Kurita R et al. PLoS One. 2013;8(3):e59890.
12. Huang X et al. Mol Ther. 2014 Feb;22(2):451-63.
13. Lee E et al. Biotechnology Journal. 2018 Apr;13(4):e1700567.

### [Disclosure]

### [Technical Problem]

An object of the present invention is to provide erythroid progenitor cells overexpressing Bcl-xL and HPV16 E6/E7 genes for mass production of RBCs, and a method of producing the same.

### [Technical Solution]

To achieve the above object, the present invention provides erythroid progenitor cells transduced with Bcl-xL and E6/E7 genes, and a method of producing the same.

The present invention also provides a recombinant vector including the Bcl-xL gene and the E6/E7 genes.

The present invention provides RBCs differentiated and matured from the erythroid progenitor cells.

### [Advantageous Effects]

The present invention provides erythroid progenitor cells for RBC production prepared through multiple transductions of expression vectors expressing Bcl-xL and HPV16 E6/E7 genes into erythroid cells. Accordingly, an erythroid progenitor cell line in which the number of cells is not reduced because cell death is reduced and proliferation of erythroid progenitor cells is maintained even during long-term culture can be established.

### [Description of Drawings]

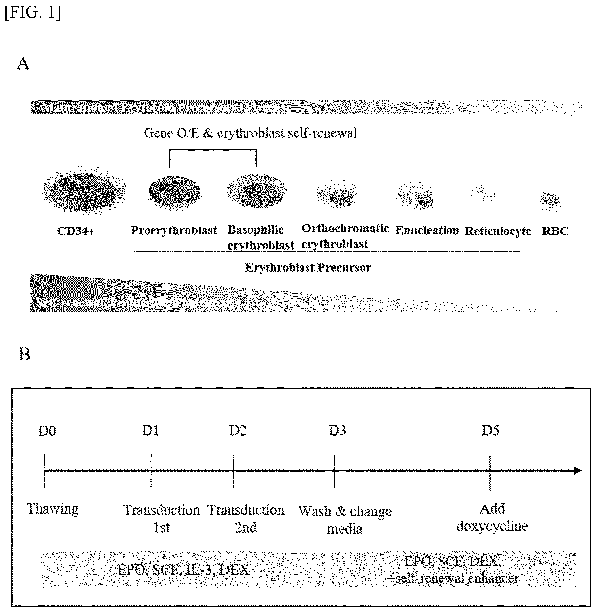

FIG. 1 schematically shows an outline for 'the erythropoesis and production of an immortalized erythroid cell line according to the present invention.

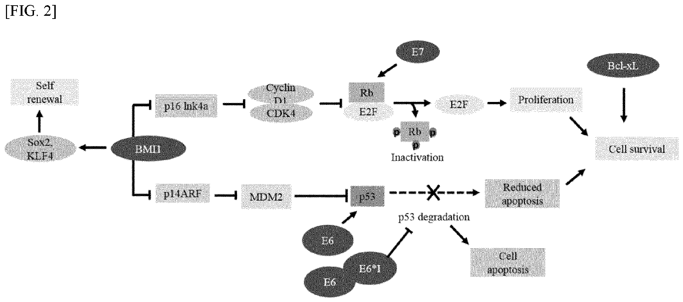

FIG. 2 shows a signaling pathway of HPV E6/E6*I and E7, BMI1, and Bcl-xL according to the present invention.

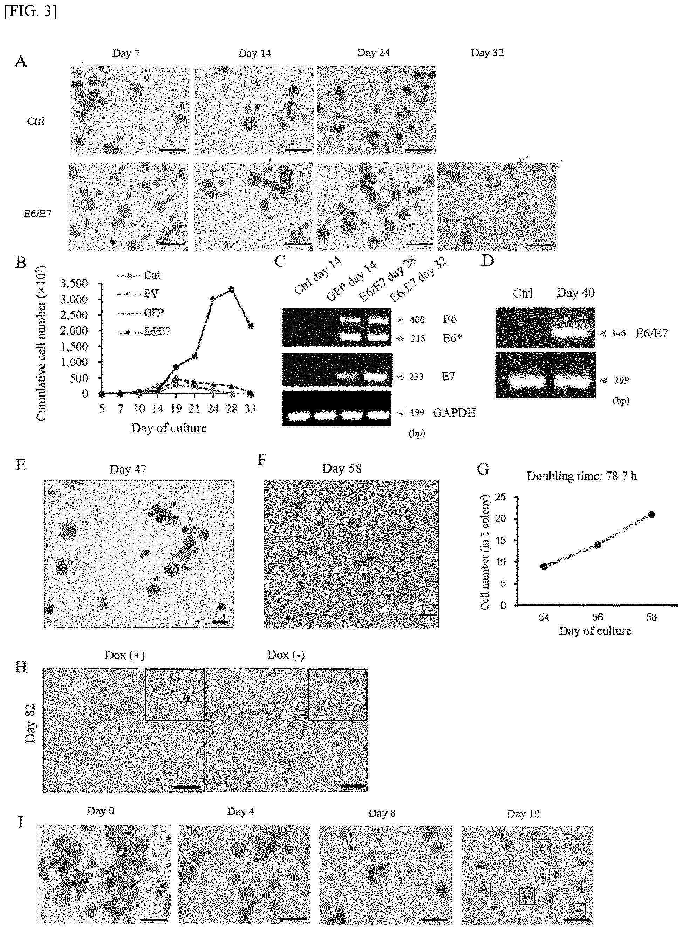

FIG. 3 shows the results of transduction of HPV E6/E7 into CD34+ cells. Non-transduced CD34+ cells were used as a control, and green fluorescent protein (GFP)-transduced cells and an empty vector (EV) were used as mock controls. (A) shows the comparison of the cell morphology of the control and transduced E6/E7 cells on days 7, 14, 24, and 32 (Red arrows; immature erythroblasts; blue arrows; orthochromatic erythroblasts). (B) shows the cumulative cell number from day 5 to day 33, and the number of cells began to decrease after day 28. (C and D) show glyceraldehyde 3-phosphate dehydrogenase (GAPDH) and E6/E7 mRNA detected by electrophoresis in transduced cells by performing RT-PCR. Full-length E6, spliced E6*I, and E7 mRNAs were detected in the control and GFP-transduced cells on day 14 and the E6/E7-transduced cells on days 28 and 32 (C) and E6/ E7 mRNA was detected in the control and E6/E7-transduced cells on day 40 (D). The size is indicated on the right (bp, base pairs). (E) shows the cell morphology in which the E6/E7 genes were transduced on day 47, and it can be confirmed that immature erythroblasts were maintained. Red arrow; immature erythroblasts. (F and G) show that the cells were tracked on day 54 of culture and proliferated on day 58. (F) shows the proliferated cells after 4 days. (G) shows the doubling time calculated by comparing the number of cells proliferated from day 54 to day 58. (H) shows the results of culturing the cells for three days under doxycycline and without doxycycline on day 79 of culture. After three days, the cells cultured without doxycycline died. (I) shows the cell morphology according to differentiation culture on days 0, 4, 8, and 10. Red; immature erythroblasts, blue; polychromatic erythroblasts, orange; orthochromatic erythroblasts, and green; red blood cells.).

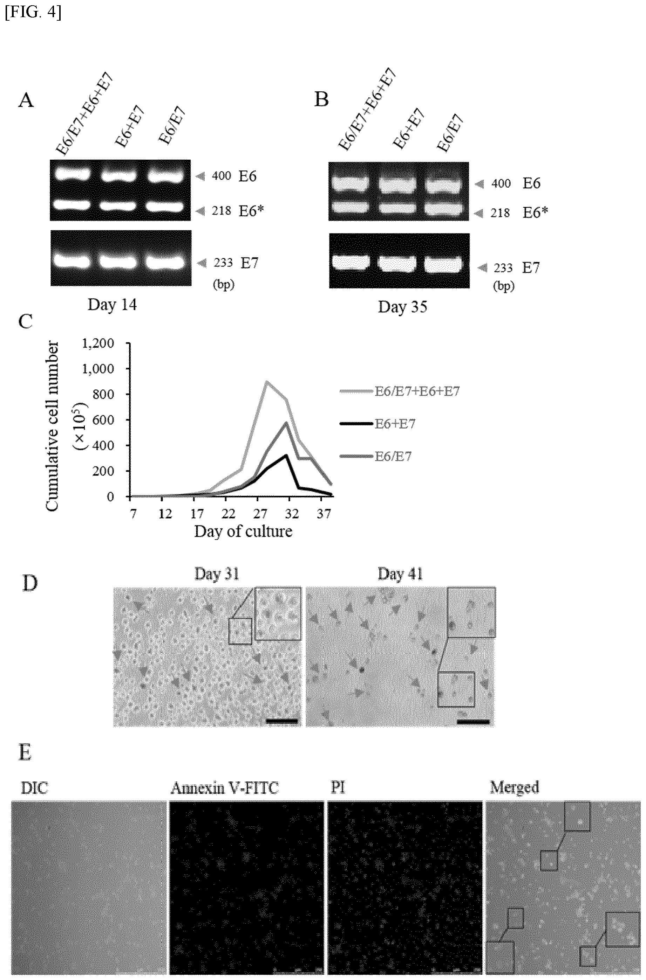

FIG. 4 (A and B) show the expression patterns of full-length E6, spliced E6*I, and E7 mRNA by RT-PCR in cells transduced with E6/E7+E6+E7, E6+E7, and E6/E7, respectively. (A) shows the results on day 14, and (B) shows the results on day 35, and the amplicons were subjected to electrophoresis. (C) shows the cumulative cell number on day 37 in cells transduced with E6/E7+E6+E7, E6+E7, and E6/E7, respectively. (D) shows senescence-associated β-galactosidase (SA-β) staining in E6/E7-transduced cells on days 14, 31, and 41. Red; SA-β stained cells, blue; dead cells. (E) shows confocal fluorescence images of E6/E7-transduced cells stained with Annexin V-FITC/PI on day 44 for cell death detection. Annexin V stains the membranes of early apoptotic cells green, and PI stains the nucleoli of late apoptotic or dead cells red.

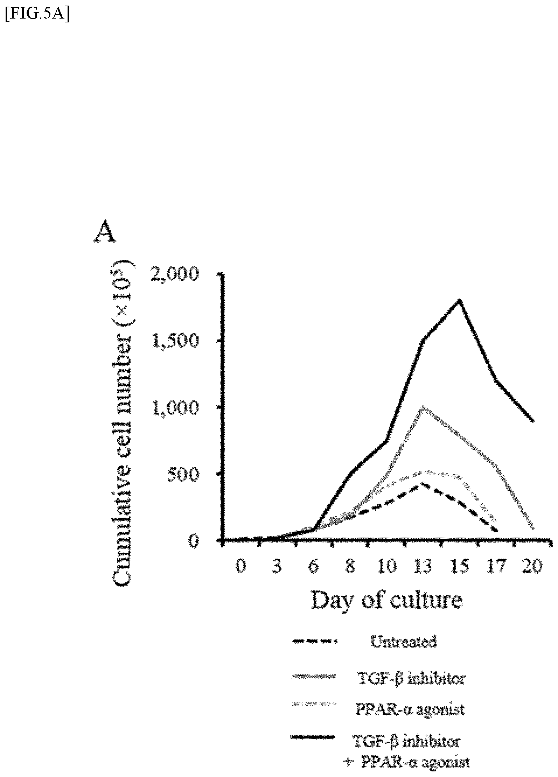

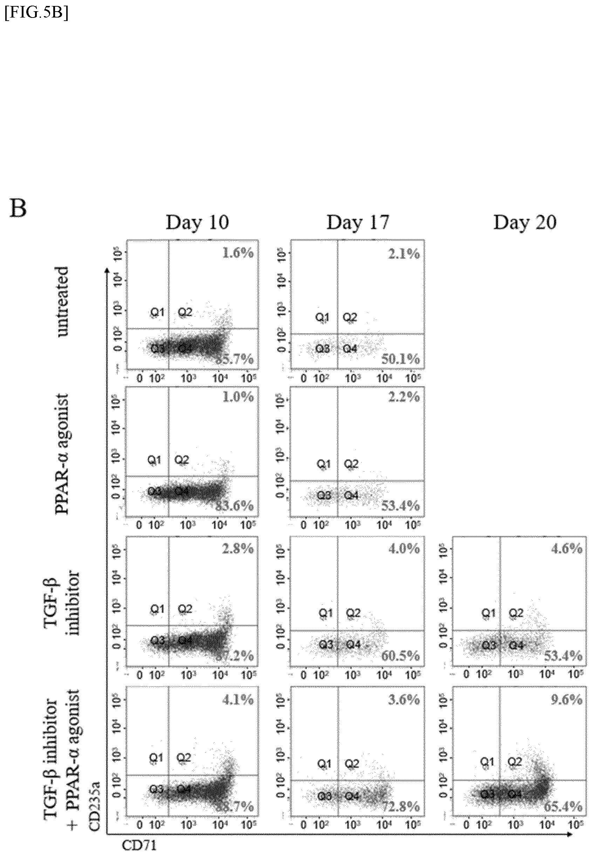

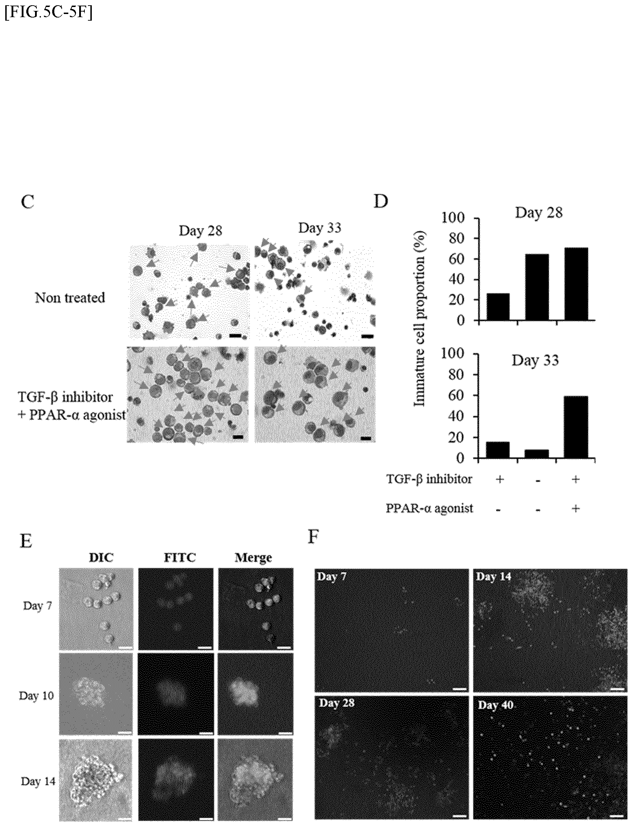

FIG. 5 (A to D) confirm the effects of a transforming growth factor (TGF)-β inhibitor and a peroxisome proliferator-activated receptor (PPAR)-α agonist on immature erythroblasts in E6/E7-transduced cells. (A) shows the cumulative cell number under various culture conditions for proliferation. Cord blood-derived CD34+ cells were grown with or without a TGF-β inhibitor and a PPAR-α agonist. (B) A flow cytometry analysis of the CD71 marker to demonstrate erythroid differentiation of cells cultured under different conditions on days 10, 17, and 20. (C and D) compare the maintained immature erythroblasts. (C) shows the cell morphology of the cells cultured with or without a TGF-β inhibitor and a PPAR-α agonist on days 28 and 33. The proportion of immature erythroblasts was assessed by cell counting after Wright-Giemsa staining. (D) In the culture using a TGF-β inhibitor and a PPAR-α agonist, the proportion of the immature erythroblasts was 60% or more. (E and F) show the assessment of the proliferation of immature erythroblasts in a similar environment within bone marrow. (E) shows the erythroblast proliferation in the presence of Matrigel on days 7, 10, and 14 of culture. Colonies of immature erythroblasts were confirmed by GFP fluorescence. (F) shows GFP fluorescence images of cells docked on fibronectin and proliferated on days 7, 14, 28, and 40 of culture.

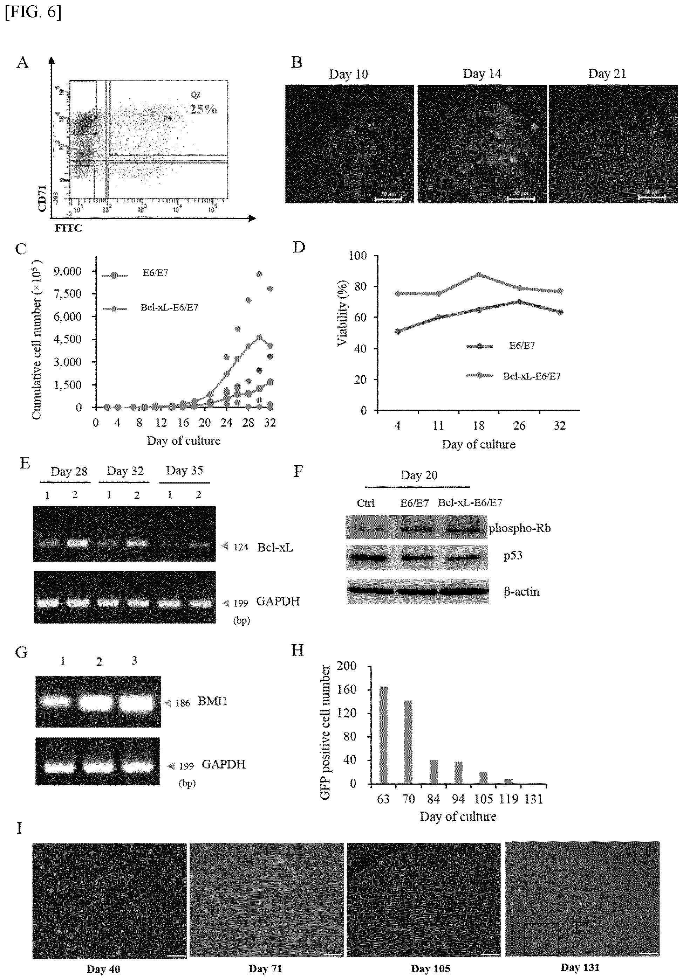

FIG. 6 shows a comparison of E6/E7- or Bcl-xL-E6/E7-transduced cells and the results of an experiment in which BMI1 was additionally transduced. (A) shows cell sorting of the transduced cells by fluorescence-activated cell sorting (FACS) on day 10. Among the transduced cells, cells expressing GFP and stained with the immature erythroid marker CD71 (FITC+/CD71+) were selected, and the FITC+/CD71+ ratio was 25%. (B) shows single cell culture by fluorescence microscopy. (C to F) show a comparison of E6/E7- or Bcl-xL-E6/E7-transduced cells. (C) shows the cumulative cell number and distribution of E6/E7 or Bcl-xL-E6/E7-transduced cells. (D) shows the viability of the cultured cells transduced with each gene. The viable cells were assessed by trypan blue staining. (E) confirms the expression of Bcl-xL in transduced cells on days 28, 32, and 35. Lane 1; E6/E7-transduced cells, Lane 2; Bcl-xL-E6/E7-transduced cells. The size is indicated on the right (bp, base pairs). (F) shows the results of a Western blot analysis of p53 and phospho-Rb. (G to I) show additional transduction of BMI1. (G) RT-PCR was performed to detect BMI1 in the transduced cells on day 33, and amplicons were subject to electrophoresis. Lane 1; control (untransduced cells), Lane 2; Bcl-xL-E6/E7+E6+E7 + BMI1, Lane 3; Bcl-xL-E6/E7 + BMI1. The size is indicated on the right (bp, base pairs). (H) shows the number of GFP-expressing cells counted among the BMI1-transduced cells. (I) shows GFP fluorescence images of BMI1-transduced cells on days 40, 71, 105, and 131.

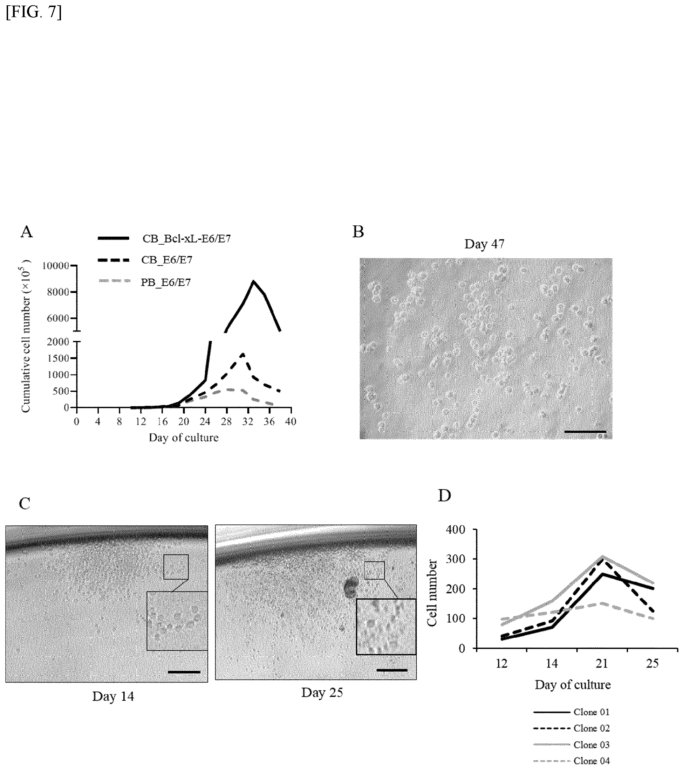

FIG. 7 relates to cell immortalization using peripheral blood or bone marrow. (A and B) show the cell culture of transduced peripheral blood CD34+ cells. (A) shows the cumulative cell number compared to CB CD34+ cells. (B) shows the proliferating cells on day 47. (C and D) show the single cell culture of transduced bone marrow CD34+ cells. (C) shows cell colonies proliferated in the single cell culture on days 14 and 25. (D) shows the cumulative cell number per clone of the single cell.

### [Modes of the Invention]

The present inventors intended to produce erythroid progenitor cell lines with higher efficiency, increase the safety of the final product by enhancing genes expressed in erythroid progenitor cells in the human body, and establish conditions for in vitro mass production of RBCs for transfusion by using a culture method that allows the proliferation of erythroid progenitor cells to be maintained even when cultured for a long period of time.

Accordingly, the present invention provides erythroid progenitor cells transduced with the Bcl-xL gene and E6/E7 genes.

In the present invention, "hematopoietic stem cells (HSCs)" refer to cells that can potentially differentiate into myeloid or erythroid lineage cells found in the blood, including RBCs, T cells, neutrophils, granulocytes, monocytes, natural killer cells, basophils, dendritic cells, eosinophils, mast cells, B cells, platelets, and megakaryocytes. For example, HSCs may be isolated from cells derived from human umbilical cord blood, peripheral blood, or bone marrow, and preferably, may be isolated from cells derived from human umbilical cord blood HSCs.

CD34 is a glycosylated transmembrane protein commonly used as a marker for blood- and bone marrow-derived primitive progenitor cells, such as HSCs and endothelial stem cells. In the present invention, the term "CD34 positive cells" refers to cells that express the CD34 protein, such as HSCs, endothelial stem cells, and mesenchymal stem cells.

In the present invention, "progenitor cells" are undifferentiated cells with the ability to self-replicate and differentiate, but are ultimately differentiated cells in which the type of cells into which they ultimately differentiate has already been determined.

In the present invention, "erythroid progenitor cells" may refer to erythroid cells before enucleation, and may be immature erythroid progenitor cells or pre-enucleation cells positive for Glycophorin A, which is an erythroid-specific molecule. For example, the erythroid progenitor cells may be selected from the group consisting of proerythroblasts, basophilic erythroblasts, polychromatic erythroblasts, and orthochromatic erythroblasts.

The present inventors confirmed that efficiency increases when genes are transduced after differentiating HSCs into erythroid cells. Since HSCs may differentiate into different lineages other than erythroid cells, there is a high possibility that erythroid lineage cell lines will be established when genes are transduced in the erythroid cell state. Therefore, in one embodiment of the present invention, gene transduction may be performed in the erythroid cell state. Specifically, in the present invention, gene transduction may be performed after differentiating HSCs into erythroid cells. Specifically, in the present invention, gene transduction may be performed when the erythroid cells are in the burst forming unit erythroid (BFU-E), colony forming unit erythroid (CFU-E), proerythroblast, or basophilic erythroblast state.

In the present invention, gene transduction includes any method of transducing a nucleic acid into an organism, cell, tissue or organ, and may be performed by selecting an appropriate standard technique depending on the host cell, as known in the art. These methods include electroporation, protoplast fusion, calcium phosphate (CaPO4) precipitation, calcium chloride (CaCl2) precipitation, agitation using silicon carbide fibers, agrobacteria-mediated transformation, polyethylene glycol (PEG), dextran sulfate, and Lipofectamine, but are not limited thereto.

In the present invention, the transduced gene may be a gene that regulates the cell cycle related to cell proliferation. Specifically, the cell proliferation-related genes may be HPV16 E6/E7 and BMI1. More preferably, the gene may be HPV16 E6/E7.

In addition, the gene transduced in the present invention may be a pro-survival gene related to inhibiting cell death. Specifically, the pro-survival gene may be Bcl-2, Bcl-xL, Bcl-w, Mcl-1, or Bfl-1. More preferably, the gene may be the Bcl-xL gene.

When only genes related to cell proliferation are transduced, there was a problem that the longer the culture is, the more the cell number decreases due to differentiation and cell death, and the proliferation of erythroid progenitor cells is not maintained. Therefore, in the present invention, by transducing a pro-survival gene along with a gene related to cell proliferation, a cell line in which cell death is inhibited, cell proliferation is enhanced, and long-term culture is possible may be provided. In an example of the present invention, it was confirmed that in an experimental group into which both a cell proliferation-related gene and a pro-survival gene were transduced, the survival rate and proliferation of cells increased compared to a group into which only a cell proliferation-related gene was transduced.

In one embodiment of the present invention, the Bcl-xL gene and the E6/E7 genes may be transduced into one vector.

In one embodiment of the present invention, the erythroid progenitor cells may be further transduced with the BMI1 gene. BMI1 is known to prevent cell aging and maintain self-renewal by inhibiting the expression of p16 and p19. In an example of the present invention, it was confirmed that long-term culture for 150 days or longer is possible when the BMI1 gene is further transduced.

The Bcl-xL gene, E6/E7 genes, and BMI1 gene used in the present invention include not only genes whose cDNA sequences have already been published, but also homologs identified in the related art based on the homology of these known cDNA sequences.

In the present invention, additional substances involved in the proliferation and maintenance of erythroid progenitor cells may be treated. For example, the erythroid progenitor cells may be cultured in a medium further containing a TGF-β inhibitor, a PPAR-α agonist, or a combination thereof. Specifically, in an example of the present invention, it was confirmed that when a TGF-β inhibitor and a PPAR-α agonist were added to a medium, the proportion of erythroid progenitor cells increased compared to the control group, as the number of days of culture increased.

In the present invention, the TGF-β inhibitor may be A-83-01, SD-208, GW788388, RepSox, LY2109761, LY2157299, LY364947, SB505124, galunisertib, vactosertib, LY364947, SB431542, SB525334, or a combination thereof, but is not limited thereto.

In the present invention, the PPAR-α agonist may be GW7647, WY14643, fenofibrate, clofibrate, gemfibrozil, bezafibrate, or a combination thereof, but is not limited thereto.

In the present invention, the TGF-β inhibitor may be contained in a medium at a concentration of 100 nM to 500 nM, 150 nM to 450 nM, 150 nM to 400 nM, or 200 nM to 300 nM. In addition, the PPAR-α agonist may be contained in the medium at a concentration of 5 µM to 20 µM, or 5 µM to 15 µM.

In addition, the present invention provides a method of producing erythroid progenitor cells, including a step of transducing a Bcl-xL gene and E6/E7 genes into erythroid cells differentiated from HSCs.

All of the above-described information regarding erythroid progenitor cells may be directly applied or applied mutatis mutandis to the method of producing erythroid progenitor cells.

In one embodiment of the present invention, the erythroid cells may be selected from the group consisting of BFU-Es, CFU-Es, proerythroblasts, and basophilic erythroblasts.

In addition, in one embodiment of the present invention, the method further includes a step of transducing a BMI1 gene.

In one embodiment of the present invention, the method further includes a step of further treating with a TGF-β inhibitor and/or a PPAR-α agonist.

The present invention provides a recombinant vector transduced with a Bcl-xL gene and E6/E7 genes. The vector may further include a BMI1 gene.

In the present invention, the term "vector" refers to a nucleic acid molecule capable of transporting another nucleic acid linked thereto. One type of vector is a "plasmid," which is a circular double-stranded DNA loop into which an additional DNA segment may be ligated. Another type of vector is a viral vector, which allows an additional DNA segment to be ligated into a viral genome. Some vectors are capable of self-replication within a host cell when transduced into the host cell (e.g., bacterial vectors having a bacterial origin of replication and episomal mammalian vectors). Other vectors (e.g., non-episomal mammalian vectors) may be integrated into a host cell's genome when transduced into the host cell and replicate along with the host genome. In addition, some vectors may direct the expression of genes to which the vectors are operably linked. In the present specification, these vectors are referred to as "recombinant expression vectors" (or simply, "expression vectors"). In general, expression vectors useful in recombinant DNA techniques are usually in the form of plasmids, and since plasmids are the most commonly used vector type, the terms "plasmid" and "vector" may be used interchangeably. However, the present invention also includes other forms of expression vectors such as viral vectors (e.g., adenovirus vectors, adeno-associated virus (AAV) vectors, herpes virus vectors, retroviral vectors, lentiviral vectors, baculovirus vectors) that provide equivalent functions. Preferably, a retroviral vector may be used. Preferably, a lentiviral vector may be used to increase transduction efficiency.

In addition, it is preferable that the vector further include a selection marker.

In the present invention, the term "selection marker" is used to facilitate selection of transformed cells into which Bcl-xL gene, HPV16 E6/E7 genes, and BMI1 gene expression cassettes are transduced. A selection marker that may be used in the vector of the present invention is not particularly limited as long as it is a gene that allows whether the vector has been transduced to be easily detected or measured, but typical examples include markers that impart selectable phenotypes such as drug resistance, auxotrophy, resistance to cytotoxic agents, or surface protein expression, for example, blasticidin, doxycycline, GFP, puromycin, neomycin (Neo), hygromycin (Hyg), histidinol dehydrogenase gene (hisD), or guanine phosphoribosyltransferase (Gpt).

The present invention also provides RBCs differentiated and matured from the above erythroid progenitor cells.

The erythroid progenitor cells of the present invention may be differentiated into mature erythroid cells by culturing them in a medium for differentiation and maturation. The medium used in the present invention may be a medium for obtaining RBCs from erythroid progenitor cells in vitro and may be a cell culture minimal medium (CCMM) containing only a carbon source, a nitrogen source, and trace element components. In other words, it is a mixture containing essential sugars, amino acids, and water necessary for cells to live, and is a basic medium excluding serum, nutrients, and various growth factors. For example, the cell culture minimal medium may be Dulbecco's Modified Eagle's Medium (DMEM), Endothelial Differentiation Medium (EDM), Minimal Essential Medium (MEM), Basal Medium Eagle (BME), Roswell Park Memorial Institute (RPMI) 1640, F-10, F-12, It may be α-MEM, Glasgow's MEM (G-MEM), or Iscove's Modified Dulbecco's Medium (IMDM).

In addition, the medium for differentiation and maturation may be a cell culture medium to which any one of thrombopoietin (TPO), interleukin (IL)-1α, IL-3, IL-4, IL-5, IL-6, IL-9, IL-11, erythropoietin (EPO), granulocyte-macrophage colony-stimulating factor (GM-CSF), stem cell factor (SCF), and granulocyte colony stimulating factor (G-CSF), Flt3 ligand, heparin, and the like, or a combination of two or more of these is added. In particular, RBCs may be cultured for about 7 to 15 days in the presence of EPO (2 to 100 U/mL, preferably about 10 U/mL) or in the presence of EPO (2 to 100 U/mL, preferably about 10 U/mL) and SCF (10 to 200 ng/mL, preferably about 50 ng/mL). The culture environment may be any suitable environment for inducing differentiation of red blood cells in vitro, but for example, culture is performed under conditions of 5% CO2 and 36 to 38 °C, preferably 37 °C.

Hereinafter, the present invention will be described in detail by examples. However, the following examples only illustrate the present invention, and the content of the present invention is not limited to the following examples.

### [Examples]

### - Experimental materials and methods

### 1. Isolation of HSCs (CD34 positive cells) derived from umbilical cord blood

The umbilical cord blood used in the present invention was received from a healthy mother who gave written consent to donate in advance, and the present invention was conducted after receiving approval from the Institutional Review Board of Hanyang University (IRB No. HYI-2019-07-006). Umbilical cord blood mononuclear cells were isolated through centrifugation using Ficoll-Hypaque. Then, CD34+ cells were isolated from the mononuclear cells using an immunomagnetic isolation method (CD34+ isolation kit (Stemcell Technologies Inc., Vancouver, Canada)).

### 2. Virus production for cell line establishment

### Lentivirus particle production and titer measurement

293FT cells were cultured in DMEM (Gibco, Grand Island, NY) containing non-heat-inactivated 10% fetal calf serum (Gibco), 0.1 mM MEM non-essential amino acids (NEAA) (Gibco), 6 mM L-glutamine (Gibco), and 500 µg/ml Geneticin (Gibco) at 37 °C in a 5% CO2 incubator (Thermo Fisher Scientific Inc.). Cryopreserved 293FT cells were thawed and used for transfection after three passages. One day before transfection, cells were cultured in a T75 flask with a culture medium without Geneticin. For transfection, Lipofectamine^{™} 2000 (Invitrogen, Carlsbad, CA) was used to transfect 293FT cells with lentiviral packaging plasmid psPAX2 (Addgene, catalog No. 12260), envelope plasmid pMD2.G (Addgene, catalog No. 12259), and a FUW-tetO lentiviral vector each containing GFP, E6/E7, Bcl-xL-E6/E7, and BMI1-GFP, and the medium was replaced with a fresh medium after 12 hours. 48 hours after transfection, the supernatant containing viral particles was collected, centrifuged (3,000 rpm, 15 min, 4 °C), and filtered through a 0.45 µm filter. The supernatant containing the produced viral particles was concentrated by ultracentrifugation (20,000 g, 4 h, 4 °C). Afterward, CD34 positive cells were seeded at 1×10⁵ cells/ml in 24 wells and then transduced with the concentrated virus using a 5-fold serial dilution method. After 24 hours, the medium was changed to a fresh medium, and on day 5 of culture, doxycycline was added for the expression of the transduced gene. After two to three days of gene expression, the level of GFP expression in the cells was measured by flow cytometry. In the case of vectors without tagged fluorescence, the titer was calculated by comparing the measured value of GFP through the Lenti-X^{™} GoStix^{™} kit (Takara Bio Inc., Otsu, Japan) with the value of the GFP virus measured by the above flow cytometry.

### 3. HSC transduction and culture

HSCs were cultured for one to three days in a medium containing StemSpan SFEM (Stemcell Technologies) with 6 IU/ml EPO (Calbiochem, San Diego, CA), 100 ng/ml SCF (R&D Systems, Minneapolis, MN), and 10 ng/ml IL-3 (R&D Systems). Then, 5×10⁴ cells were transduced with the virus concentrated in the above culture medium and polybrene (8 µg/ml, Sigma). The multiplicity of infection (MOI) of the virus during transduction was calculated to be 15 to 30. 24 hours after the transduction, the medium was replaced with a fresh virus-free medium, and from day 5 of culture, the StemSpan SFEM medium was supplemented with 3 IU/ml EPO, 50 ng/ml SCF, 10⁻⁶ M dexamethasone, and 250 nM TGF-β inhibitor (LY2157299, PeproTech, Rocky Hill, NJ), 10 µM PPAR-α agonist (GW7647, Sigma), and 1 µg/ml doxycycline (Takara Bio Inc.) to induce selective expression of HPV16 E6/E7 in the transduced cells. The efficiency of transduction was confirmed on day 7 of culture through GFP fluorescence expression of GFP-transduced cells, and the cells were maintained at a concentration of 2 to 3×10⁵ cells/ml while replacing the medium with a fresh medium every two to three days. In addition, to obtain the cell proliferation and survival rate, the cells were stained with trypan blue (Gibco) and counted.

To differentiate the transduced cells into RBCs, the following procedure was followed. From day 0 to day 6 of differentiation, heat-activated 5% fetal bovine serum, 6 IU/ml EPO, 100 ng/ml SCF, 10 ng/ml IL-3, 10⁻⁶ M dexamethasone, and 1 µg/ml doxycycline were added to IMDM (Gibco) to culture the cells. From day 6 of differentiation, the cells were differentiated in IMDM supplemented with 500 mg/ml transferrin, 6 IU/ml EPO, and 10⁻⁶ M dexamethasone.

To form an in vivo intramedullary environment, cells were cultured by attaching Matrigel (Corning Inc., NY) and fibronectin (Takara Bio Inc.) to a plate. For 3D culture, Matrigel was diluted to 8 mg/ml in the culture medium. The diluted Matrigel was spread on the plate and allowed to stand at 37 °C for 30 minutes. The cells were cultured on the plate on which Matrigel was spread. Next, a fibronectin-coated plate was attached to the plate at a concentration of 5 µg/cm2 at room temperature for two hours or at 4 °C for 24 hours. Afterward, the fibronectin solution was removed, and the cells were subjected to a reaction with 2% bovine serum albumin (BSA) at room temperature for 30 minutes and washed with phosphate-buffered saline (PBS) before use.

### 4. Flow cytometry (FCM)/Fluorescence-activated cell sorting (FACS)

The level of GFP expression in the transduced cells was measured using a flow cytometer CantoII (BD Biosciences Inc., San Jose, CA) to measure the transduction efficiency and lentivirus titer. In addition, a fluorescence-activated cell sorter FACS Aria II (BD Biosciences Inc.) was used to sort the transduced erythroid cells by attaching an erythroid marker to the transduced cells.

The following antibodies were used: glycophorin A (GPA; CD235a)-FITC (Life Technologies Inc., Gaithersburg, MD); and CD71-PE (BioLegend Inc., San Diego, CA). The cells were washed with 0.1% BSA, then antibodies were added and stained for 15 minutes at 4 °C, while blocking light. In addition, a fluorescence microscope (Nikon Eclipse Ti; Nikon Corporation) was used to confirm the GFP fluorescence expressed in the cells cultured in the plate.

### 5. Cellular aging and death analysis

To analyze cell aging and death, the cells were collected during culture, and senescence-associated reagents and death-measuring antibodies were attached to them. The following reagents and antibodies were used: a Senescence cell histochemical staining kit (Sigma); and an Annexin V-FITC/PI detection kit (BioLegend Inc.).

To confirm cell aging after 30 days, when cell proliferation began to decrease during long-term culture, the cells were washed with PBS (Gibco) and fixed with 2% formaldehyde for seven minutes at room temperature. Next, after washing three times with PBS, the senescence-associated staining reagent was added to the cell culture medium, and the cells were cultured in a 37 °C incubator (Thermo Fisher Scientific) for 24 hours without injecting CO2, and the stained cells were confirmed under a microscope.

Next, to confirm cell death, the cells were washed with 0.1% BSA, antibodies were added, light was blocked, and the cells were stained for 15 minutes at room temperature. The cells to which antibodies were attached were smeared on a slide glass, and the degree of cell death was confirmed using a confocal microscope TCS SP5 (Confocal microscope; Leica Microsystems Inc., Vienna, Austria).

### 6. Cell morphology analysis

Some of the cells in culture were spread on a slide using a cell centrifuge (Cellspin; Hanil Science Industrial Co. Ltd., Incheon, Korea) and stained with a Wright-Giemsa stain (Sigma), and the state, shape, and maturity of the cells were confirmed in a blind manner. Cell photos were taken using an optical microscope (Nikon Eclipse TE2000-U; Nikon Corporation).

### 7. PCR analysis

RNA was isolated from the cells using Trizol Reagent (Ambion Inc., Austin, USA) according to the specific number of culture days, and the concentration of RNA was measured using a spectrophotometer (Nanodrop 2000, Thermo Fisher Scientific). After synthesizing cDNA using a SuperScript III First-Strand Synthesis System (Invitrogen), mRNA expression was measured in duplicate through real-time (RT)-PCR using SYBR green (TOPreal qPCR 2X PreMIX; Enzynomics Co. Ltd., Daejeon, Korea). The values were corrected using GAPDH, and the gene expression levels of HPV16 E6, HPV16 E7, HPV16 E6/E7, Bcl-xL, and BMI1 overexpressed through transduction were confirmed. The primer sequences used in the experiment are as follows (Table 1).

**[Table 1]**

| | Forward primer | Reverse primer |
|---|---|---|
| GAPDH | 5'-GAAGGTGAAGGTCGGAGTC- 3' (SEQ ID NO: 1) | 5'-GACAAGCTTCCCGTTCTCAG-3' (SEQ ID NO: 2) |
| HPV16 E6 | 5'-GCGACCCAGAAAGTTACCAC- 3' (SEQ ID NO: 3) | 5'-GCAACAAGACATACATCGCCGG- 3' (SEQ ID NO: 4) |
| HPV16 E7 | 5'- GCAACCAGAGACAACTGATCTC -3' (SEQ ID NO: 5) | 5'-TGGGGCACACAATTCCTAGTG-3' (SEQ ID NO: 6) |
| HPV16 E6/E7 | 5'- GTCAAAAGCCACTGTGTCCTG- 3' (SEQ ID NO: 7) | 5'-GCACAACCGAAGCGTAGAGT-3' (SEQ ID NO: 8) |
| Bcl-xL | 5'- GAGCTGGTGGTTGACTTTCTC-3' (SEQ ID NO: 9) | 5'-TCCATCTCCGATTCAGTCCCT-3' (SEQ ID NO: 10) |
| BMI1 | 5'-CCAGGGCTTTTCAAAAATGA- 3' (SEQ ID NO: 11) | 5'-CCGATCCAATCTGTTCTGGT-3' (SEQ ID NO: 12) |

### - Results

### 1. Establishment of erythroid progenitor cell line

In the present invention, CD34+ cells derived from umbilical cord blood, peripheral blood, and bone marrow were immortalized by transduction of the HPV16 E6/E7 genes. Since continuous expression of the gene in the transduced erythroid progenitor cells does not lead to differentiation into RBCs, which are the final product, an attempt was made to express the HPV16 E6/E7 genes using a Tet-inducible expression system that allows selective expression. (FIG. 1B).

After transduction, the state and maturity of the cells were confirmed through Wright-Giemsa staining. As a result of staining the non-transduced control group, on day 14 of culture, the immature erythroblasts were still dividing, but differentiated cells were confirmed. On day 24 of culture, the culture was terminated as mature polychromatic erythroblasts formed a majority. In the transduced control group, the majority of immature erythroblasts were proliferating until day 32 of culture, confirming the difference in the cell state between the control group and the experimental group in which the gene was overexpressed by culture day (FIG. 3A). In addition, with regard to the proliferation rate, the cells increased over 613-fold in the non-transduced control group until day 17 of culture, and then gradually decreased until the culture was terminated on day 24 of culture. In the case of the experimental group overexpressing E6/E7, the proliferation was 641-fold on day 17, which was similar to the control group, but unlike the control group, where culture was terminated on day 24, cells overexpressing E6/E7 proliferated over 3,300-fold in culture after 28 days of culture, and the cells gradually decreased from day 31 of culture (FIG. 3B). In terms of cell proliferation, the effect of E6/E7 was confirmed as the proliferation was at least 5.5 times more than that of the non-transduced control group. However, with regard to the number of days of cell culture, contrary to the expectation that overexpression of E6/E7 would enable long-term culture through proliferation by a continuous cell cycle, and thus immortalization could be achieved, from day 30 of culture, proliferation decreased, the number of cells decreased, and many differentiated cells appeared. Therefore, to find problems that appeared after 30 days of culture, first, an experiment was performed to confirm the presence or absence of E6/E7 gene expression in the transduced cells and the function of the vector.

### 1.1 Confirmation of expression of HPV16 E6/E7 genes

To confirm the expression of E6/E7 genes in the transduced cells, mRNA was extracted, and reverse transcription PCR was performed. Normal erythroid progenitor cells cultured for 14 days as a control group, cells transduced only with GFP, and cells overexpressing the corresponding genes were collected on days 28 and 32 of culture, and E6 and E7 genes were each identified. As a result, the expression of E6 and E7 genes was not confirmed in the normal erythroid progenitor cells and the GFP-transduced cells, and in the transduced cells cultured for 28 days and 32 days, the expression of the full-length form and the spliced form of E6 was observed, and it was confirmed that the full-length form of E6 increased in the cells cultured for 32 days compared to the cells cultured for 28 days. It was confirmed that E7 was also not expressed in the normal erythroid progenitor cells and the GFP-transduced cells but expressed in the transduced cells cultured for 28 and 32 days. It was found that the expression of E7 increased in the cells cultured for 32 days compared to the cells cultured for 28 days (FIG. 3D). In addition, the mRNA from the cells cultured for 40 days was collected to confirm the total mRNA expression of E6/E7, and as a result, it was confirmed that E6/E7 was expressed (FIG. 3C). The above results showed that E6/E7 genes were well expressed in the transduced cells even after 30 days.

### 1.2 Long-term culture effect of HPV16 E6/E7 genes

It was attempted to confirm that the cells in long-term culture were due to the effect of the transduced E6/E7 genes through the cells that survived and proliferated despite the cell death due to apoptosis and the cell death due to differentiation after 30 days of culture. First, the cell state and maturity were confirmed by Wright-Giemsa staining. As a result of collecting cells on day 47 of culture and performing Wright-Giemsa staining, it was confirmed that immature erythroblasts were proliferating and that the immature erythroblast form was maintained even in the middle stage of the culture (FIG. 3E). On day 54 of culture, proliferating cells were observed for four days to calculate the doubling time based on the number of divisions. It was found that the cells were proliferating from 9 cells on day 54 of culture to 21 cells after 4 days, and the measured doubling time was to 78.7 hours (FIGS. 3F and 3G). In addition, to confirm whether the Tet-on system of the vector was operating normally during long-term culture, cells on day 79 of culture were cultured for three days in a medium without doxycycline. As a result, in the control group cultured after removing doxycycline, cells were viable and proliferating, but in the experimental group cultured after removing doxycycline, cells were found to be dead (FIG. 3H). Since the removal of doxycycline from the proliferation medium rather than the differentiation medium led to cell death without differentiation into RBCs, it was necessary to confirm whether RBCs could be produced by culturing in a differentiation medium. First, because changes in the composition of the medium from the proliferation medium, in which the transduced cells were being cultured for a long period of time, to the differentiation medium may affect the cells, doxycycline was added to the differentiation medium for five days. Afterward, from day 6 of differentiation, differentiation was performed without doxycycline, and the cell morphology was analyzed by Wright-Giemsa staining. The day when the medium was changed to the differentiation medium was set to be day 0 when the majority was immature erythroblasts. On day 4 of differentiation, immature erythroblasts were still proliferating by doxycycline, and cells differentiated into polychromatic erythroblasts were also observed. On day 8 of differentiation, almost no immature erythroblasts were seen, and many polychromatic erythroblasts were observed. On day 10 of differentiation, polychromatic erythroblasts as well as RBCs were observed, and many orthochromatic erythroblasts were observed (FIG. 3I).

Through these results, it was confirmed that the Tet-on system operates normally even in long-term culture, and cells survive due to the effect of the HPV16 E6/E7 genes expressed by the vector. In addition, it was confirmed that final differentiation of RBCs could be achieved by removing doxycycline through the differentiation medium.

### 1.3 Confirmation of E6 splicing and cell death

It is known that HPV E6/E7 is polycistronic, so when E6/E7 are expressed from the same promoter, various patterns of E6 mRNA are expressed through alternative splicing, and at this time, the function of spliced E6*I interferes with the existing E6, which inhibits p53. Therefore, it was attempted to determine whether the amount of the spliced form and the full-length form of E6 expressed in the transduced cells have an effect on cell line production. In addition to the previously constructed E6/E7 vectors expressed from the same promoter, an E6 vector and an E7 vector was separately produced, and it was expected that the full-length form of E6 would be expressed in a large quantity. Transduction was attempted by dividing into E6/E7+E6+E7, E6+E7, and E6/E7 experimental groups. The mRNA from the cells cultured for 14 and 35 days after transduction was collected, and differences in expression of the spliced and full-length forms of E6 and E7 were confirmed through RT-PCR. First, as a result of PCR of the cells cultured for 14 days, it was confirmed that the difference in the expression level between the full-length form and the spliced form of E6 was small in both experimental groups, and that the expression levels of the full-length form and spliced form of E6 in the E6 + E7 experimental group were lower than those of the other two experimental groups (FIG. 4A). In addition, in the cells cultured for 35 days, the full-length and spliced forms of E6 were expressed similarly, and the expression levels in the E6+E7 experimental group was lower than those of the other experimental groups. It was found that the expression of E7 was not significantly different among all the experimental groups on days 14 and 35 (FIG. 4B).

The proliferation rates of the transduced cells in each experimental group were compared. It was confirmed that the proliferation in the E6/E7+E6+E7 experimental group was the most at 900-fold by day 28, the proliferation in the E6/E7 experimental group was 577-fold by day 31, and the proliferation in the E6+E7 experimental group was the least at 320-fold by day 31 (FIG. 4C). Through this, it was confirmed that there was no significant difference in the expression patterns of the full-length and spliced forms of E6 mRNA in the E6+E7 experimental group, where the full-length form was expected to be expressed in a large quantity, compared to the other two experimental groups, and the degree of splicing according to the promoter was negligible. Since the E6+E7 experimental group exhibited the lowest proliferation rate, it was concluded that the inhibition of the E6 function is not significantly affected by splicing.

Based on the above results, it was confirmed that E6/E7 was expressed in long-term culture of the transduced cells, and it was confirmed that the maintenance and proliferation of immature erythroblasts was due to the effect of the E6/E7 genes. Compared to 21 days for typical RBC culture, the proliferation period was extended by two or three times or more, and long-term culture having an average of 57 days and a maximum of 150 days was possible. However, although the E6/E7 genes were continuously expressed in all cultures, long-term culture could not be achieved for 60 days or longer, and regardless of the E6 mRNA expression pattern, the number of cells tended to decrease as the culture continued in most of the experiments. To confirm the state of the cells after 30 days when the proliferation rate began to decrease, staining with senescence-associated (SA)-β galactosidase, which is a senescence marker, was performed. It was confirmed that among the cells cultured for 31 days, the number of SA-β gal-stained cells increased, and among the cells cultured for 41 days, most of the viable cells were SA-β-stained cells (FIG. 4D). After three days, the cells cultured for 44 days were stained with Annexin V/propidium iodide (PI) and then examined with confocal laser microscopy (CLSM) to determine the degree of cell death (apoptosis). As a result of the staining, cells stained only with Annexin V, which were in the early stage of apoptosis, and cells stained with both Annexin V and PI, which were in the late stage of apoptosis and cell necrosis, were observed (FIG. 4E). Through this, it was concluded that even though E6/E7 was expressed, cells did not die rapidly after day 30, but rather passed through the senescence stage for several days before cell death.

Therefore, in addition to the E6/E7 genes, it was attempted to find better culture conditions than the current culture environment. The following experiment was conducted to find the optimal conditions for culturing an erythroid progenitor cell line with the goal of reducing the increase in cell death during culture and prolonging the proliferation period of immature erythroblasts having high self-proliferation ability.

### 2. Study of erythroid progenitor cell line culture conditions

When producing a cell line, with the goal of immortalizing immature erythroblasts with high self-dividing and proliferative abilities, the proliferation of immature erythroblasts in the initial culture after transduction may be increased to increase the proportion of immature erythroblasts that are immortalized. For this, substances that could increase the proliferation of immature erythroblasts were searched for. In addition, it is known that hematopoietic stem progenitor cells proliferate in the bone marrow in vivo and maintain their self-proliferative ability through various actions. Through this, it was expected that when an environment similar to the bone marrow is created in vitro and culture is performed therein to continuously maintain immature erythroblasts, the self-proliferation ability of immature erythroblasts would be maintained, which could be helpful in cell line culture in addition to the genetic effect of transduction.

### 2.1 Proliferation of immature erythroblasts

There is an TGF-β inhibitor (LY2157299) which is known to be effective in the maintenance and proliferation of immature erythroblasts, and a PPAR-α agonist (GW7647) that may have a synergistic effect on the proliferation of immature erythroblasts with glucocorticoid was explored, and an experiment was performed on the effects of the two substances. First, the two substances were added to a typical CD34+ cell culture under different conditions and cultured. In terms of cell proliferation, the cells proliferated the most by 1,500-fold by day 15 of culture when the culture was performed by adding the two substances together, and the cells proliferated by 1,000-fold by day 13 of culture in the experimental group where only the TGF-β inhibitor was added. Next, it was confirmed that there was no significant difference between the experimental group to which only the PPAR-α agonist was added and the control group to which nothing was added because their proliferation rate was 500-fold and 400-fold, respectively (FIG. 5A). In addition, the immature erythroblast marker CD71 was attached to the proliferating cells, and the proportion of immature erythroblasts was confirmed through flow cytometry. The proportion of immature erythroblasts in the control group, where culture was terminated after 17 days, and in the experimental group, which was treated only with the PPAR-α agonist, was 50% and 53%, respectively, and the highest values were found to be 72% and 65%, respectively, on day 17 and day 20 in the experimental group where both substances were added together (FIG. 5B). Through this, it was confirmed that adding the self-proliferation inducer TGF-β inhibitor and PPAR-α agonist together was helpful in the proliferation and maintenance of immature erythroblasts. Therefore, to see the above effects in transduced cell culture, the following experiment was performed. The cell morphology was confirmed and the proportion of immature erythroblasts was confirmed on days 28 and 33 of culture in the experimental group where a self-proliferation inducer was added and the control group where a self-proliferation inducer was not added. As a result, immature erythroblasts were found to be 65% of all cells on day 28 in the control group and 7.7% on day 33, and when treated with only TGF-β inhibitors, they were 26.7% of all cells on day 28 of culture and 15.2% on day 33 of culture. As a result of culturing with both a TGF-β inhibitor and a PPAR-α agonist, it was confirmed that the proportion of immature erythroblasts was an average of 71.5% on day 28 and 60% on day 33 (FIGS. 5C and 5D). Through the above results, a significant difference in the proliferation and immature erythroblast proportion was found in the experimental group cultured with both a TGF-β inhibitor and a PPAR-α agonist, and it was also concluded that treating with the two substances in an early stage of culture when immature erythroblasts proliferate a lot could be helpful.

In order to create an environment similar to the bone marrow in an in vitro culture, according to a 3D structure where proliferation may occur with the interactions with VLA-4 expressed on the erythroblast cell surface, which is the environment of immature erythroblasts that proliferate in the bone marrow in vivo, cultivation was attempted using Matrigel and fibronectin, which are substances capable of interacting with the VLA-4 of immature erythroblasts and enabling 3D culture. When cultured in Matrigel, cells were observed on day 7, and it was confirmed that the cells proliferated in the form of a colony, which is a characteristic of immature erythroblast proliferation, until day 14. However, after day 14, the cells failed to proliferate beyond a certain number, and the culture was terminated, and although the clumped colonies were released and culture was attempted again, they failed to proliferate (FIG. 5E). Therefore, it was confirmed that the conditions using Matrigel were not very helpful in producing a cell line. In the fibronectin culture, cells were relatively widely distributed on a fibronectin-coated culture dish on day 7. One week later, on day 14, it was seen that the cells proliferated a lot and GFP fluorescence was also expressed a lot. It was confirmed that it was possible to culture more than a certain number of cells, and the fibronectin culture is helpful in maintaining immature erythroblasts and extending the proliferation period (FIG. 5F). However, after day 30, the number of GFP-expressing cells began to decrease, and the number of cells did not increase. As a result of confirming GFP fluorescence on day 40 of culture, it was seen that the number of fluorescent cells was significantly reduced.

It was attempted to extend the cell proliferation and culture days by maintaining the self-proliferation ability of immature erythroblasts by improving the culture conditions and environment of immature erythroblasts. The above results confirmed that the use of substances that are helpful for the proliferation and maintenance of immature erythroblasts and the creation of an environment similar to the bone marrow help to prolong the proliferation of immature erythroblasts compared to the general RBC culture, but the number of cells decreased, and cell death was also not resolved.

### 2.2 Study to reduce cell death

As problems in cell line production, the cell death that occurs during the culture process and the substances secreted from apoptotic cells affect the surrounding normal cells so that they also undergo cell death, which was thought to be the cause of the decrease in proliferation rate due to cell death. To exclude this apoptotic effect, it was attempted to classify and culture only immature erythroblasts among the transduced cells. First, CD71, an immature erythroblast marker, was attached to the transduced cells, and an analysis was performed by fluorescence-activated cell sorting to sort the transduced cells exhibiting GFP fluorescence and including attached CD71 (GFP+/CD71+). The proportion of the GFP+/CD71+ cells was confirmed to be 25% (FIG. 6A). A comparison was made between the single cell culture method, in which homogeneous colonies may be formed by distributing the sorted cells one cell per well in a 96-well culture plate, and the existing culture conditions in which a constant cell number concentration was maintained. As a result, in the single cell culture method, cells proliferated a lot after 10 days of culture, and GFP fluorescence expression was also confirmed. After 4 days, on day 14 of culture, it was found that the number of cells increased, but the cell size decreased, and some cells were undergoing cell death. On day 21 of culture, the results showed that GFP fluorescence was not expressed and the cells did not proliferate, and the culture was terminated (FIG. 6B). Through the single cell culture method, cell death was relatively reduced and cell proliferation occurred, but the number of cells did not tend to increase beyond a certain number. Compared to the culture conditions in which a constant cell number concentration was maintained, with regard to the number of days of culture, the concentration-maintained culture showed proliferation for 40 days or more on average, but in the single cell culture, the culture was terminated after an average of 20 days. Therefore, it was concluded that the single cell culture method is not of great help, and maintaining a constant cell number concentration would be important for cell proliferation.

In addition, besides the culture method, it was attempted to reduce cell death by adding genes that can be helpful in cell line production along with the E6/E7 genes to a vector. First, it was expected that Bcl-xL, a pro-survival gene which is expressed as an endogenous gene, may exhibit a synergistic effect through overexpression with the existing E6/E7 genes to reduce cell death observed in the culture of transduced immature erythroblasts, thereby helping cell survival (FIG. 2). HPV16 E6/E7 and Bcl-xL were included together in one vector by adding the Bcl-xL gene to the previously constructed lentiviral vector, and a comparison was made with cells transduced with existing HPV16 E6/E7 only. As a result, cells transduced with only the E6/E7 genes proliferated 1,687-fold on average, and the most proliferated cells exhibited an increase of 3,355-fold. Cells additionally transduced with the Bcl-xL gene proliferated an average of 4,640-fold, and the largest increase was 8,800-fold. It was confirmed that the average difference was 2.7-fold from E6/E7 (FIG. 6C). The cell viability was maintained at 70% or less in the case of the cells expressing only the E6/E7 genes from day 4 to day 32 of culture, and the cell viability of the cells additionally transduced with Bcl-xL was maintained at 75% or more (FIG. 6D). The mRNA of the cells transduced with E6/E7 and Bcl-xL-E6/E7 was collected on days 28, 32, and 35 of culture to compare the expression of Bcl-xL. Although Bcl-xL is an endogenous gene that is expressed without overexpression, it was confirmed that Bcl-xL was overexpressed more in the experimental group additionally transduced with Bcl-xL than in the cells transduced with only E6/E7. However, it was found that as the culture progressed, Bcl-x expression, both endogenous expression and overexpression, decreased (FIG. 6E). In addition, to check the activities of E6 and E7, it was attempted to identify p53 and pRb proteins, respectively, through a Western blot analysis. It was found that p53 decreased in the E6/E7 experimental group additionally transduced with Bcl-xL, and it was confirmed that p53 also decreased in the E6/E7 experimental group compared to the control group. It was found that pRb increased in the two experimental groups compared to the control group (FIG. 6F).

Through the above results, it was found that the addition of Bcl-xL was effective in the early proliferation rate and cell survival rate, but the problem of cell number decrease due to cell death when the culture progressed over a long period of time was not resolved.

Referring to a previous study in which, through an mRNA microarray analysis at each stage of erythroid differentiation, BMI1, whose expression was increased in the proliferation of erythroid progenitor cells and is related to self-proliferation ability, was selected and overexpressed, each gene was transduced in addition to Bcl-xL-E6/E7 in an attempt to produce a cell line. In the present study, to conduct an experiment by additionally transducing BMI1 at the time when cell death increases and cell survival rate decreases, transduction was carried out on days 26 and 27 of culture. GFP was tagged to the BMI1 vector, and five days after transduction, the degree of transduction was confirmed through GFP fluorescence, and mRNA was collected to confirm the overexpression of the BMI1 gene (FIG. 6G). In the cell culture with added BMI1, cells that survived for 131 days were found, and GFP fluorescence confirmed that the number of GFP-expressing cells decreased from the middle stage to the late stage of the culture, but cells expressing GFP until day 131 were confirmed (FIGS. 6H and 6I). The transduction of the BMI1 gene allowed for long-term culture for four months or longer, but did not show a significant effect on cell proliferation.

Through the above experiment, additional genetic manipulation to extend the proliferation period of immature erythroblasts and reduce cell death exhibited a significant difference in cell proliferation of immature erythroblasts in the early stage compared to the control group, but the problem of proliferation during long-term culture was not resolved.

### 3. Production of cell lines using peripheral blood and bone marrow cells

In addition to cord blood, it was attempted to produce a cell line using CD34+ cells derived from peripheral blood and bone marrow. First, an experiment was conducted in three ways: by transducing a gene into monocytes isolated from peripheral blood, by inducing the monocytes to differentiate into immature erythroblasts for four to five days and then performing transduction, and by isolating CD34+ cells from peripheral blood and performing transduction. The same transduction method was used for peripheral blood as for umbilical cord blood, but as a result of culture, proliferation was not achieved significantly, and there was a significant difference in cell proliferation compared to umbilical cord blood. As a result of transducing the cells isolated from the peripheral blood with Bcl-xL-E6/E7, the proliferation was 550-fold on day 28 of culture, and when cells isolated from cord blood were transduced with E6/E7 and Bcl-xL-E6/E7, the proliferation was 1,600-fold and 8,800-fold, respectively, and so it was confirmed that there was a difference of 2.9-fold and 5.5-fold (FIG. 7A). With regard to the proliferation days, proliferation occurred for an average of 32 days, and culture was possible for up to 50 days. It was confirmed that the cells continued to proliferate on day 47 of culture (FIG. 7B).

Since the number of cells decreased significantly after transduction of E6/E7 into bone marrow-derived CD34+, single cell culture was performed. As a result, proliferation was observed from day 14 to day 21 of culture and began to decrease after day 21, and it was confirmed that many cells had died after 25 days of culture (FIGS. 7C and 7D). In the case of bone marrow-derived CD34+, it was confirmed that most cultures were terminated before day 30, and culture for up to 35 days was possible.

The present invention has been described with reference to the above-described examples and experimental examples, but these are merely illustrative, and those skilled in the art will understand that various modifications and other equivalent examples and experimental examples are possible therefrom. Therefore, the true scope of technical protection of the present invention should be determined by the technical spirit of the attached claims.

## Claims

1. An erythroid progenitor cell transduced with a Bcl-xL gene and E6/E7 genes.

2. The erythroid progenitor cell of claim 1, wherein the transduction is performed in an erythroid cell in a burst forming unit erythroid (BFU-E), colony forming unit erythroid (CFU-E), proerythroblast, or basophilic erythroblast state.

3. The erythroid progenitor cell of claim 1, wherein the erythroid progenitor cell is derived from a hematopoietic stem cell.

4. The erythroid progenitor cell of claim 1, wherein the Bcl-xL gene and the E6/E7 genes are transduced into one vector.

5. The erythroid progenitor cell of claim 1, wherein a BMI1 gene is further transduced

6. The erythroid progenitor cell of claim 1, wherein the erythroid progenitor cell is further treated with a transforming growth factor (TGF)-β inhibitor and a peroxisome proliferator-activated receptor (PPAR)-α agonist.

7. A method of producing an erythroid progenitor cell, comprising transducing a Bcl-xL gene and E6/E7 genes into an erythroid cell differentiated from a hematopoietic stem cell.

8. The method of claim 7, wherein the erythroid cell is selected from the group consisting of a burst forming unit erythroid (BFU-E), a colony forming unit erythroid (CFU-E), a proerythroblast, and a basophilic erythroblast.

9. The method of claim 7, further comprising transducing a BMI1 gene.

10. The method of claim 7, further comprising further treating with a TGF-β inhibitor and/or a PPAR-α agonist.

11. A recombinant vector comprising a Bcl-xL gene and E6/E7 genes.

12. The recombinant vector of claim 11, further comprising a BMI1 gene.

13. A red blood cell differentiated and matured from the erythroid progenitor cell of claim 1.
